**Europäisches Patentamt**

(19) **European Patent Office**

**Office européen des brevets**

(11) Publication number : **0 277 096 B1**

(12) # EUROPEAN PATENT SPECIFICATION

(45) Date of publication of patent specification :
08.07.92 Bulletin 92/28

(51) Int. Cl.⁵ : **A61K 37/547, A61L 15/16, C12N 9/96**

(21) Application number : **88810030.2**

(22) Date of filing : **22.01.88**

(54) **Improved thrombin preparations.**

The file contains technical information submitted after the application was filed and not included in this specification

(30) Priority : **28.01.87 US 7555**

(43) Date of publication of application :
**03.08.88 Bulletin 88/31**

(45) Publication of the grant of the patent :
**08.07.92 Bulletin 92/28**

(84) Designated Contracting States :
**BE CH DE FR GB IT LI LU NL SE**

(56) References cited :
**EP-A- 0 221 700
FR-A- 912 350
GB-A- 2 081 090
US-A- 2 433 299**

(73) Proprietor : **WARNER-LAMBERT COMPANY
201 Tabor Road
Morris Plains New Jersey 07950 (US)**

(72) Inventor : **Silbering, Steven Bernard
112-41 72nd Road
Forest Hills New York 11375 (US)**
Inventor : **Blythe, Rowland Parker, Jr.
5563 Cherokee Drive
Washington Michigan 48094 (US)**
Inventor : **Fawzi, Mahdi Bakir
11 Timberline Drive
Flanders N.J. 07836 (US)**
Inventor : **Nesbitt, Russell Ulmer
292 Millen Avenue
Somerville, N.J. 08876 (US)**

(74) Representative : **Silbiger, Jakob, Dr.
c/o CAPSUGEL AG Fabrikmattenweg 2-4
CH-4144 Arlesheim (CH)**

## Description

Thrombin, a proteolytic enzyme, is essential for hemostasis. It is a principal reagent in the formation of blood clots via fibrin production. Due to its effectiveness as a clotting aid, thrombin and its preparations are useful during surgical procedures to control bleeding. While dry thrombin is available, liquid preparations are generally preferred due to handling and time considerations.

Until now, there have been no highly stable liquid thrombin preparations which are both storage stable and ready for use during surgery. This is because thrombin, when dissolved in water or saline, rapidly loses its activity due to denaturation and autolysis of the thrombin protein.

It has been discovered that sterile, storage stable thrombin preparations can be produced by adding to purified thrombin, in a suitable medium, stabilizing quantities of certain buffers. Optionally, saline and one or more polyol stabilizers can also be employed.

This invention is related to that disclosed in U.S.SN. 791,836, filed October 28, 1985 (US-A-4696812).

In one preferred embodiment, a solution containing 2,000 U/ml (units per milliliter) purified thrombin in 0.45 M. NaCl solution containing 50% (v/v) glycerol and 0.05 $\underline{M}$ phosphate buffer, pH 6.0, was prepared. This solution, after storage at room temperature (25°C) for 6 months, had retained all of its original clotting activity. After storage for 12 months at room temperature, this solution still retained 80% of its original activity.

In another preferred embodiment, a composition containing 1500 u/ml of purified thrombin with 0.05 $\underline{M}$ acetate buffer, 2.7% (0.45 $\underline{M}$) NaCl and 25% glycerol at pH 5.0 showed 86% activity, based on fibrometric readings, after 6 months storage at 25°C.

These preferred compositions showed even better activity retention when stored at 4°C, ie., 100% for the phosphate system after 12 months and 95% for the acetate system after 12 months.

The thrombin compositions and methods of the invention have several advantages over conventional preparations and methods for assisting in blood clotting.

Unlike powdered preparations, the compositions of the instant invention require no reconstitution prior to use. Thus, measuring, mixing or sterilizing of one or more component(s) or container(s) are not important considerations. The instant preparations can be used with only minimal, ie., little or no, preparation before final use.

Furthermore, the stability of the instant thrombin-containing materials is such that the need for stock inventories and/or rotation of products is largely eliminated. Unlike most saline or water-solutions of thrombin, which are stable for only about 1 week at 4°C, the instant preparations are designed to be stable at normal refrigeration temperatures (ie., 4°C) and at room temperature (ie., 25°C) for 6 months or more.

It is known that high concentrations of glycerol, sucrose, and other polyols can stabilize proteins in solution. In the case of thrombin, it is known that a glycerol concentration of 67% can greatly stabilize a 1,000 u/ml thrombin solution. However, use of high glycerol concentrations is not practical in the large scale manufacture of a sterile thrombin solution because of the high viscosity of such a preparation. The instant compositions, which contain 50% or less, of glycerol avoid these problems.

Other advantages and aspects of the invention will become apparent from a consideration of the following description of the invention.

The invention concerns, in its broadest aspects:

I. Thrombin compositions, preferably solutions, which contain purified thrombin, a polyol, and at least one buffer.

II. Coagulant products useful as dressings which contain the preparations of I.

III. Methods of making the thrombin compositions of I.

The preparations made in accordance with the invention must contain, in a liquid medium, substantially purified thrombin, and one or more of the buffers of the invention. They may contain saline, and other substances conventionally employed in protein preparations.

While the term "preparations" is employed, it should be noted that applicants contemplate all types of formulations in which thrombin, in substantially solubilized or highly dispersed form, is present in combination with one or more of the instant glycols and buffers.

Liquid preparations are generally preferred. Solutions of thrombin are highly preferred. When a liquid formulation is made, it is generally preferred that the solvent(s) or other diluent(s) employed have a suitable miscibility with thrombin such that production standards, e.g., uniformity of thrombin concentration from batch to batch, can be readily met.

The thrombin employed is a purified thrombin obtained by two-stage ion-exchange chromatography. In the first stage, DEAE Sepharose® (which is a diethylaminoethyl Sepharose) is employed to adsorb a large portion of the non-thrombin protein. In the second stage, CM-Sepharose® (which is a carboxyethyl Sepharose) is employed to adsorb thrombin and allow the remaining non-thrombin protein to pass through the column. Both DEAE-Sepharose and CM-Sepharose are ion exchange materials used for liquid chromatography separations. They are supplied by Pharmacia, Inc., Laboratory Separation Division, 800 Centennial Avenue, Piscataway, New Jersey, 08854. The thrombin is eluted from the column by using either 0.05 $\underline{M}$ acetate in 0.45 $\underline{M}$ NaCl, pH 5.0 as by using 0.05 $\underline{M}$ phosphate in 0.45 $\underline{M}$ NaCl, pH 6.0. This thrombin solution is then mixed with glycerol containing either acetate buffer or phosphate buffer and saline, in order to prepare the stabilized solution described as the invention.

Thrombin is known to be soluble in physiological saline -- i.e., a solution containing 0.9% NaCl in water. However, other saline solutions are contemplated as useful herein. Furthermore, the replacement of all or part of the NaCl in such solutions with one or more other suitable salts is contemplated.

Water is a preferred medium for the preparations of the invention. However, the use of one or more other diluents which do not adversely affect the solubility and/or stability of thrombin in the subject preparations is desirable.

One such diluent is glycerol. Other useful polyols include mannitol, sorbitol, sucrose and glucose. Mixtures are operable. Glycerol is highly preferred.

The glycerol or other polyol ingredient(s) will be employed at a total concentration of from about 10 to about 60 wt. %, preferably 25 to 50 wt % based on total composition weight.

Unless stated otherwise, all quantities recited are weight percentages based on total compositions weight.

Suitable buffer systems are those whose aqueous solutions will maintain the pH of the final thrombin solution between 5.0 and 8.0, with a preferred pH range of 5.0 to 6.0. It is highly preferred that when a phosphate buffer is used the final pH of the preparation be 6.0 to 6.5 and when an acetate buffer is used, the final pH be 5.0.

pH measurements are made using an ordinary pH meter with a combination electrode.

Useful buffer systems include acetate, phosphate, succinate, bicarbonate, imidazole, TRIS, and the zwitterionic buffers described by N.E. Good and S. Izawa, in Methods in Enzymol, 24, Part B, 53 (1972); and W. F. Ferguson, K. I. Braunschweiger, W. R. Braunschweiger, J. R. Smith, J. McCormick, C. C. Wasmann, N. P. Jarvis, D. H. Bell and N. E. Good in Anal. Biochem, 104, 300 (1980).

Suitable reagents for use in the instant buffer systems include MES, ACES, BES, MOPS, TES, HEPES and the like. Phosphate should only be used when calcium ion is absent or in the presence of EDTA. Mixtures of such reagents can be employed. If mixed buffers are used, the final pH should be suitably adjusted.

Buffers containing phosphate ion and acetate ions are preferred. Mixtures are operable.

The buffer systems disclosed in U.S.S.N. 06/791,836 can also be employed herein.

The buffers will be present in the buffer solution, along with water and/or other suitable diluent(s) at total concentrations of 0.02 $\underline{M}$ to 1$\underline{M}$, preferably 0.05 $\underline{M}$ to 0.10 $\underline{M}$.

The use of various other conventional additives, e.g., antioxidants, colorants and surfactants, is also contemplated. Glutathione may be employed as an optional ingredient. Amino acids may be employed as optional ingredients, but their presence must not be in such quantities as to interfere with the stabilizing action of the polyol and buffer components on the purified thrombin. In general, it is preferred that they be used in only minute quantities at concentrations of 0.5% or less, if at all.

In general, the concentration ranges for the ingredients discussed above will be within the limits set out in Table I. Percentages are based on total composition weight.

## Table I

| Ingredient | Composition | | |
| --- | --- | --- | --- |
| | Broad | Preferred | Highly Preferred |
| Thrombin units/ml | 10-10,000 | 50-5,000 | 500-2,000 |
| Buffer solution ($\underline{M}$) | 0.01-1.0 | 0.02-0.2 | 0.05-0.10 |
| Diluent/Solvent | - | - | - |
| Polyol | 0-50 | 10-50 | 25-50 |

Hemostatic materials, such as GELFOAM®, SURGICEL®, and AVICEL®, and collagen which are presently used alone or in combination with thrombin powder or thrombin in saline, can be effectively used with the stabilized thrombin formulations of the present invention using a variety of techniques. Preferably, the stabilized solution is absorbed onto the hemostatic agent and the pad is freeze-dried and packaged in a sterile manner.

Antimicrobial or antibiotic agents can also be incorporated into such pads, especially for use on burn patients, where prevention of infection is critical. In addition, surfactants and salts other than NaCl can be employed. When one or more of such additives are present, their concentrations are generally within the ranges set out in Table II.

## Table II

| Additive | Broad | Weight Percentage | |
| | | Preferred | Highly Preferred |
| --- | --- | --- | --- |
| Surfactants | 0-2 | 0-0.5 | 0-0.2 |
| Antioxidants | 0-1 | 0-0.2 | 0-0.1 |
| Antimicrobials | 0-1 | 0-0.5 | 0-0.2 |
| Other additives eg., salts | 0-5 | 0-3 | 0-1 |

One type of bandage suitable in the preparation of coagulants in accordance with the invention is set forth in U.S. Patent US-A-4,363,319,

### Production of Thrombin Preparations

Crude thrombin, preferably of bovine origin, is purified by the two-stage ion-exchange chromatography described above. The purified thrombin is eluted from CM-Sepharose® by addition of 0.05 $\underline{M}$ phosphate in 0.45 $\underline{M}$ sodium chloride pH 6.0, or by addition of 0.05 $\underline{M}$ acetate, pH 5.0 in 0.45 $\underline{M}$ sodium chloride. The thrombin eluted from the column is highly concentrated and, in order to prepare the thrombin preparation of the present invention, must be diluted. This is done by, for example, addition of a volume of a solution containing 50% (w/w) glycerol in 0.05 $\underline{M}$ phosphate, 0.45 $\underline{M}$ sodium chloride, pH 6.0, to an equal volume of eluted thrombin in phosphate-buffered saline. The resulting thrombin preparation contains the desired concentration of purified thrombin in 25% glycerol, 0.05 $\underline{M}$ phosphate, 0.45 $\underline{M}$ sodium chloride. This particular preparation has a pH of approximately 6.0.

The resulting preparation is assayed by measurement of clotting time using a BBL fibrometer.

### Examples

The following examples will serve to better illustrate the invention.

### Example I

The preparation of phosphate-buffered thrombin composition is given above. An acetate-buffered composition would be prepared in a similar manner, by substituting 0.05 $\underline{M}$ acetate, pH 5.0, for 0.05 $\underline{M}$ phosphate, pH 6.0.

Thrombin compositions of the present invention are assayed by the following procedure:

The thrombin activity levels are determined by measurement of clotting time on a BBL fibrometer. The source of fibrinogen is pooled human plasma diluted 1:1 with 0.9% saline. The thrombin solution is diluted 200-fold with 0.5% polyethylene glycol 8000 in imidazole buffered saline. Into a coagulation cup is added 0.2 ml of diluted plasma. This is kept at 37°C for 3 minutes, and to this solution is added 0.1 ml of diluted thrombin solution, which has also been kept at 37°C for 3 minutes. Clotting time is determined directly from the fibrometer reading. The number of thrombin units/ml remaining is determined from a standard curve of thrombin concentration vs. clotting time.

### Example II

The data in TABLE III illustrates the stability of purified thrombin in a glycerol-phosphate system, pH 6.0.

Table III Solution Stability of Purified Thrombin (2000 Units/ml) at 4°and 25°C in Various Glycerol-Phosphate (0.45 M NaCl, 0.05 M Phosphate, pH 6.0) Formulations.

| | Storage | | Test Formulation % of Activity | | | |
|---|---|---|---|---|---|---|
| Temp. (C°) | Time (Months) | | A | B | C | D |
| | | Glycerol | 50% (V/V) | 25% | 10% | - |
| 25 | 0 | | 100 | 100 | 100 | 100 |
| | 1 | | 93 | 98 | - | - |
| | 2 | | 87 | 96 | - | - |
| | 3 | | 104 | 96 | - | - |
| | 4 | | - | - | 45 | 20 |
| | 6 | | 105 | 81 | - | - |
| | 12 | | 80 | 25 | - | - |
| 4 | 0 | | 100 | 100 | 100 | 100 |
| | 1 | | 92 | 108 | 82 | 88 |
| | 2 | | 90 | 110 | 87 | 78 |
| | 3 | | 88 | 109 | 96 | 79 |
| | 6 | | 106 | 115 | 101 | 62 |
| | 12 | | 106 | * | 71 | 27 |

*Contaminated

It should be noted that thrombin stability is not maintained at room temperature when the level of glycerol is lower than 25%.

Example III

The data in TABLE IV illustrates the stability of purified thrombin in a glycerol-acetate system, pH 5.0.

Table IV -

Solution Stability of Purified Thrombin (1500 Units/ml) at 4°and 25°C in 25% Glycerol-Acetate Formulations.

| | Storage | Test Formulation | |
|---|---|---|---|
| | | _% of Activity_ | |
| | | _A_ | _B_ |
| | (Months) | | |
| | (NaCl) | 2.7% | 2.7% |
| | (ACETATE) | (0.45 _M_) | (0.45 _M_) |
| | | 0.05 _M_ | 0.10 _M_ |
| | (pH) | 5.0 | 5.0 |
| TEMP | _Time_ | | |
| (C°) | (Months) | | |
| 4 | 0 | 100 | 100 |
| | 0.25 | 93 | 94 |
| | 1 | 94 | 91 |
| | 2 | 102 | 108 |
| | 3 | 102 | 102 |
| | 6 | 100 | 110 |
| | 12 | 95 | 96 |
| 25 | 0 | 100 | 100 |
| | 0.25 | 99 | 97 |
| | 1 | 102 | 102 |
| | 2 | 98 | 98 |
| | 3 | 89 | 87 |
| | 6 | 86 | 91 |
| | 12 | 56 | 57 |

It is instructive to compare the stabilities of impure and purified thrombins in similar formulations. A solution of impure thrombin (1500 units/ml in 25% (w/w) glycerol, 0.05 $\underline{M}$ acetate, 0.45 $\underline{M}$ NaCl, having a pH of 5.0, when stored at 25°C for 1 month, was found to retain 68% of its initial clotting activity. A similar formulation, containing 1500 units/ml purified thrombin, was found to retain 100% of its initial activity after 1 month at 25°C, and was found to retain 86% of its activity after 6 months at 25°C.

Example IV

Preparation of a hemostat.

A GELFOAM® pad is saturated with a solution of purified thrombin in 0.05 $\underline{M}$ acetate buffer, pH 5.0, containing 0.45 $\underline{M}$ saline. The pad is freeze-dried. Thrombin contained in such a dry pad can maintain its stability for prolonged periods, even at room temperature. It was found that pads of GELFOAM®, saturated with purified thrombin in glycerol/acetate, and stored in the wet state, completely disintegrated in 6 days at room temperature.

**Claims**

1. A stable thrombin composition containing
(a) purified thrombin,
(b) a polyol, and a buffer which contains either acetate or phosphate ions, wherein the preparation has a pH of 5.0 to 6.0.

2. The composition of claim 1 wherein the buffer contain phosphate ions and the pH is 6.0 to 6.5.

3. The composition of claim 1 wherein the buffer contains acetate ions and the pH is 5.0 to 5.5.

4. The composition according to anyone of the claims 1 to 3 wherein the polyol is glycerol.

5. A hemostat for use as a wound dressing comprising the composition as claimed in anyone of the claims 1 to 4 and a substrate.

6. A method of making a product comprising a stabilized preparation comprising the step of contacting purified thrombin with a polyol and a buffer wherein the product has a final pH of 5.0 to 6.0.

7. The method of claim 6 wherein the buffer is phosphate buffer and the final pH is 6.0 to 6.5.

8. The method of claim 6 wherein the buffer is an acetate buffer and the final pH is 5.0 to 5.5.


**Patentansprüche**

1. Stabile Thrombinmasse, enthaltend
(a) gereinigtes Thrombin,
(b) ein Polyol und einen Puffer mit entweder Acetat- oder Phosphationen, wobei die Zubereitung einen pH-Wert von 5,0 bis 6,0 aufweist.

2. Masse nach Anspruch 1, dadurch gekennzeichnet, daß der Puffer Phosphationen enthält und der pH-Wert 6,0 bis 6,5 beträgt.

3. Masse nach Anspruch 1, dadurch gekennzeichnet, daß der Puffer Acetationen enthält und der pH-Wert 5,0 bis 5,5 beträgt.

4. Masse nach einem der Ansprüche 1 bis 3, dadurch gekennzeichnet, daß das Polyol aus Glycerin besteht.

5. Hämostat zur Verwendung als Wundverband, umfassend die Masse entsprechend einem der Ansprüche 1 bis 4 und ein Substrat.

6. Verfahren zur Herstellung eines eine stabilisierte Zubereitung umfassenden Produkts durch Inberührungbringen von gereinigtem Thrombin mit einem Polyol und einem Puffer, wobei das Produkt einen End-pH-Wert von 5,0 bis 6,0 aufweist.

7. Verfahren nach Anspruch 6, dadurch gekennzeichnet, daß es sich bei dem Puffer um einen Phosphatpuffer handelt und der End-pH-Wert 6,0 bis 6,5 beträgt.

8. Verfahren nach Anspruch 6, dadurch gekennzeichnet, daß es sich bei dem Puffer um einen Acetatpuffer handelt und der End-pH-Wert 5,0 bis 5,5 beträgt.


**Revendications**

1. Une composition stable de thrombine contenant :
(a) de la thrombine purifiée,
(b) un polyol et un tampon contenant des ions acétate ou phosphate, la préparation ayant un pH de 5,0 à 6,0.

2. La composition selon la revendication 1, dans laquelle le tampon contient des ions phosphate, le pH étant de 6,0 à 6,5.

3. La composition selon la revendication 1, dans laquelle le tampon contient des ions acétate, le pH étant de 5,0 à 5,5.

4. La composition selon l'une quelconque des revendications 1 à 3, dans laquelle le polyol est le glycérol.

5. Un hémostatique destiné à être utilisé comme pansement pour blessures, comprenant la composition selon l'une quelconque des revendications 1 à 4, et un substrat.

6. Un procédé de fabrication d'un produit comprenant une préparation stabilisée, qui comprend l'étape consistant à mettre en contact de la thrombine purifiée avec un polyol et un tampon, le produit ayant un pH final de 5,0 à 6,0.

7. Le procédé selon la revendication 6, dans lequel le tampon est un tampon phosphate et le pH final est de 6,0 à 6,5.

8. Le procédé selon la revendication 6, dans lequel le tampon est un tampon acétate et le pH final est de 5,0 à 5,5.